(19) Europäisches Patentamt
European Patent Office
Office européen des brevets

(11) Numéro de publication : **0 281 449 B1**

(12) # FASCICULE DE BREVET EUROPEEN

(45) Date de publication du fascicule du brevet : **29.05.91 Bulletin 91/22**

(51) Int. Cl.[5] : **C07D 339/06**, H01L 29/28

(21) Numéro de dépôt : **88400295.7**

(22) Date de dépôt : **09.02.88**

(54) **Composés organiques du type tétrathiafulvalène utilisables pour l'élaboration de films de Langmuir-Blodgett conducteurs et leur procédé de fabrication.**

(30) Priorité : **13.02.87 FR 8701869**

(43) Date de publication de la demande : **07.09.88 Bulletin 88/36**

(45) Mention de la délivrance du brevet : **29.05.91 Bulletin 91/22**

(84) Etats contractants désignés : **DE GB NL**

(56) Documents cités :
**TETRAHEDRON, vol. 40, no. 10, 1984, pages 1817-1822, Pergamon Press Ltd, GB; A. SOUIZI et al.: "Précurseurs de nouveaux tétrathiafulvalènes: alcoylthio-4 amino-2 dithioles-1,3 et les alcoylthio-4 alcoxy-2 dithioles-1,3 préparés à partir des dithioles mésoioniques"**
**ZHURNAL OBSHCHEI KHIMII, vol. 56, no. 5, mai 1986, pages 1157-1160, Plenum Publishing Corp.; V. YU. KHODORKOVSKII et al.: "Influence of substituents on the electron-donor properties of tetrathiafulvalenes"**

(73) Titulaire : **THOMSON-CSF**
**51, Esplanade du Général de Gaulle**
**F-92800 Puteaux (FR)**

(72) Inventeur : **Robin, Philippe**
**THOMSON-CSF SCPI 19, avenue de Messine**
**F-75008 Paris (FR)**
Inventeur : **Robert, Albert**
**THOMSON-CSF SCPI 19, avenue de Messine**
**F-75008 Paris (FR)**
Inventeur : **Bertho, Françoise**
**THOMSON-CSF SCPI 19, avenue de Messine**
**F-75008 Paris (FR)**
Inventeur : **Batail, Patrick**
**THOMSON-CSF SCPI 19, avenue de Messine**
**F-75008 Paris (FR)**

(74) Mandataire : **Turlèque, Clotilde et al**
**THOMSON-CSF SCPI**
**F-92045 PARIS LA DEFENSE CEDEX 67 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 281 449 B1

Jouve, 18, rue Saint-Denis, 75001 PARIS

## Description

La présente invention se rapporte à des composés organiques du type tétrathiafulvalène utilisables pour l'élaboration de films de Langmuir-Blodgett conducteurs.

La méthode de Langmuir-Blodgett permet d'obtenir à la surface d'un liquide des couches monomoléculaires organisées de très faible épaisseur (de 10 à 40 angströms), de les transférer et de les superposer sur un substrat. Les possibilités d'applications de ces films dérivent des caractéristiques suivantes : simplicité du procédé d'élaboration, très grande précision des épaisseur obtenues (quelques angströms), orientation des molécules, propriétés physiques ajustables.

Les films de Langmuir-Blodgett sont obtenus à partir de molécules amphiphiles. Ces molécules sont allongées et présentent à l'une de leurs extrémités un groupe hydrophile et à l'autre extrémité un groupe hydrophobe. Les molécules amphiphiles les plus simples sont les acides gras $CH_3-(CH_2)_n-COOH$ qui sont constitués d'une tête polaire $-COOH$ hydrophile et d'une chaîne aliphatique hydrophobe $CH_3-(CH_2)_n$. L'acide béhénique $CH_3-(CH_2)_{20}-COOH$ est une des molécules les plus couramment utilisées.

La très faible épaisseur de ces films les rend particulièrement intéressants dans le domaine de la microélectronique. On a donc cherché à les rendre conducteurs de l'électricité. EP 193 362 décrit de tels films conducteurs réalisés à partir de tétracyanoquinodiméthane (TCNQ) convenablement substitué pour pouvoir être déposé sous forme de film de Langmuir-Blodgett.

Un autre type de conductivité serait du plus haut intérêt dans le domaine de l'électronique : la conductivité anisotrope, surtout lorsque l'anisotropie est telle que la conduction se fasse dans la direction du plan du film alors que l'isolation est assurée dans une direction perpendiculaire à ce plan.

Des matériaux organiques présentant une anisotropie de conduction sont déjà connus. Les tétrathiafulvalènes (TTF) sont des composés organiques donneurs d'électrons qui peuvent donner lieu à la formation de complexes par transfert de charges avec des accepteurs d'électrons. De tels complexes, connus sous le nom de "métaux organiques", présentent la propriété remarquable de conduire le courant électrique selon une direction privilégiée. Compte-tenu de l'intérêt de ces composés, en particulier dans le domaine de l'électronique, de très nombreux travaux ont été consacrés à l'étude de leurs synthèses et de leurs propriétés (M. NARITA, C.U. PITTMAN Jr. Synthesis 1976, p. 489 ; J.B. TORRANCE, Account Chemical Research 1979, n° 12, p. 79; F. WUDL, Account Chemical Research 1984, n° 17, p. 227). Ces molécules ne sont cependant pas amphiphiles et ne peuvent donc pas conduire à la formation de films de Langmuir-Blodgett. A ce jour, aucune méthode de préparation de molécules TTF amphiphiles n'est connue.

Des dérivés de TTF sont aussi décrits dans les documents suivants :

– Tetrahedron, vol. 40, n° 10, 1984, pages 1817-1822, Pargamon Press Ltd, GB ; A. SOUIZI et al. "Précurseurs de nouveaux tétrathiafulvalènes : alcoylthio-4 amino- 2 dithioles-1,3 et les alcoylthio-4 alcoxy-2 dithioles-1,3 préparés à partir des dithioles mésoioniques".

– Zhurnal Obshchei Khimii, vol. 56, n° 5, mai 1986, pages 1157-1160, Plenum Publishing Corp. ; V. YU. KHODORKOVSKII ET al. : "Influence of substituents on the electron-donor properties of tetrathiafulvalenes".

Afin de remédier au problème relatif à la préparation de molécules TTF amphiphiles, l'invention propose de nouvelles molécules de tétrathiafulvalène disubstituées de manière à les rendre amphiphiles. On peut ainsi obtenir des films minces de Langmuir-Blodgett qui deviennent conducteurs lorsqu'ils sont dopés par des accepteurs d'électrons (l'iode, le brome, le pentafluorure d'arsenic, etc...). De plus, la structure en couches de ces films entraîne une anisotropie de conductivité : ils sont conducteurs dans les directions du plan du film et isolants dans la direction perpendiculaire à ce plan.

L'invention a donc pour objet un composé organique du type tétrathiafulvalène, caractérisé en ce qu'il est rendu amphiphile par substitution de radicaux, le composé répondant aux formules suivantes.

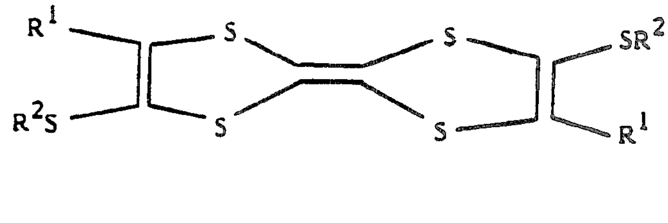

ou

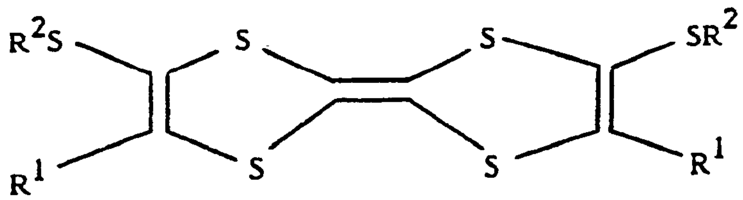

$R^1$ et $R^2$ répondant à l'une des conditions suivantes :
- $R^1$ est un groupe hydrophile et $R^2$ un groupe hydrophobe,
- $R^1$ est un groupe hydrophobe et $R^2$ un groupe hydrophile,
- $R^1$ est un groupe aromatique et $R^2$ est un groupe possédant une longue chaîne carbonée de nature hydrophobe et une extrémité hydrophile.

L'invention a aussi pour objet le procédé de fabrication de ce composé organique.

L'invention a encore pour objet un film de Langmuir-Blodgett caractérisé en ce qu'il est formé à partir du composé organique ci-dessus, le film étant rendu conducteur par dopage, par exemple à partir d'éléments chimiques accepteurs d'électrons.

L'invention sera mieux comprise et d'autres avantages apparaîtront au moyen de la description qui va suivre, donnée à titre non limitatif, et des dessins annexés parmi lesquels :
- les figures 1 et 2 représentent deux configurations possibles pour les molécules selon l'invention,
- la figure 3 illustre le procédé général de synthèse des molécules selon l'invention,
- la figure 4 représente une molécule TTF selon l'invention.

Les nouvelles molécules selon l'invention répondent aux formules chimiques générales représentées aux figures 1 (forme trans) et 2 (forme cis). Dans l'une ou l'autre de ces formules, on peut avoir :
- $R^1$ représente un groupe hydrophile et $R^2$ un groupe hydrophobe,
- $R^1$ représente un groupe hydrophobe et $R^2$ un groupe hydrophile,
- $R^1$ représente un groupe aromatique et $R^2$ possède une longue chaîne carbonée de nature hydrophobe et une extrémité hydrophile.

Le procédé mis en oeuvre pour préparer ces nouveaux TTF permet de les substituer à la fois par des groupes hydrophiles et par des groupes hydrophobes. Ceci conduit à des molécules amphiphiles très bien adaptées à l'élaboration de films de Langmuir-Blodgett.

## PROCEDE GENERAL DE SYNTHESE

Le procédé général de synthèse est représenté à la figure 3. Il comprend les étapes suivantes :
- étape (a) : préparation d'un éthylénique par action du malonitrile $CH_2(CN)_2$ sur un aldéhyde comportant le radical $R^1$,
- étape (b) : préparation d'un époxyde par action de l'hypochlorite de sodium sur le produit obtenu à l'étape (a),
- étape (c) : préparation d'un amide par action du dithiocarbamate de pipéridinium sur le produit obtenu à l'étape (b),
- étape (d) : transformation en acide carboxylique du produit obtenu à l'étape (c) de préférence par action de l'acide sulfurique,
- étapes (e) et (f), l'étape (e) ne nécessitant pas d'être isolée : on fait agir sur l'acide obtenu à l'étape (d) un mélange constitué d'anhydride acétique, de triéthylamine et de sulfure de carbone et on obtient le dithiole mésoïonique,
- étape (g) : préparation d'un sel de dithiolium par action d'un dérivé halogéné comportant le radical $R^2$ et un halogène X sur le produit obtenu à l'issue de l'étape (f),
- étape (h) : préparation de l'éthoxydithiole par action du borohydrure de sodium et de l'acide chlorhydrique sur le produit obtenu à l'issue de l'étape (g),
- étape (i) : préparation du composé TTF selon l'invention par action de l'acide trichloroacétique $CCl_3CO_2H$ sur le produit obtenu à l'étape (h).

## EXEMPLE DE SYNTHESE D'UNE MOLECULE

A titre d'exemple, on va décrire plus précisément la préparation d'une molécule particulière pour laquelle les radicaux $R^1$ et $R^2$ sont les suivants :

$R^1 = pCO_2HC_6H_4$
$R^2 = (CH_2)_{17}CH_3$

• étape (a)

Une suspension constituée de 0,1 mole de malonitrile, 200 $cm^3$ de dioxanne et 0,1 mole de 4-carboxybenzaldéhyde est refroidie dans de la glace puis additionnée de 7 $cm^3$ de pipéridine. Après 40 mn d'agitation, la solution est diluée par 100 $cm^3$ d'eau glacée, acidifiée à l'acide chlorhydrique 6N puis on procède à une extraction à l'éther. Après évaporation du solvant, l'éthylénique est obtenu de façon quantitative.

• étape (b)

A 10 g de l'éthylénique obtenu à l'issue de l'étape (a), en suspension dans 120 $cm^3$ d'acétonitrile, on ajoute 120 $cm^3$ d'hypoclorite de sodium 2,3 N sous agitation et en maintenant le pH aux environs de 6 par addition d'acide sulfurique 2 N. Après un temps de 10 mn on ajoute 200 $cm^3$ d'eau glacée et on extrait le milieu par de l'éther. L'évaporation de l'éther conduit à l'époxyde dont le point de fusion est 214°C.

• étape (c)

A une suspension de $6.10^{-3}$ mole de dithiocarbamate de pipéridinium dans 15 $cm^3$ de tétrahydrofuranne sec, on ajoute goutte à goutte, sous agitation et sous atmosphère d'azote, $7.10^{-3}$ mole de l'époxyde obtenu à l'issue de l'étape (b) en solution dans 15 $cm^3$ de tétrahydrofuranne sec. Après 15 heures à la température ambiante, on acidifie le milieu par l'acide chlorhydrique 6 N. Le précipité formé est lavé à l'eau et séché. L'amide obtenu a un point de fusion de 208°C.

• étape (d)

5,5 g de l'amide obtenu précédemment sont mis en solution dans 60 $cm^3$ d'acide sulfurique. Après 2 heures à la température ambiante, on dilue par de la glace jusqu'à précipitation complète de l'acide désiré dont le point de fusion est 205°C.

• étape (e) et (f)

Ces étapes amènent à la synthèse du dithiole mésoïnique. Ce composé peut être obtenu directement à partir de l'acide obtenu à l'issue de l'étape (d) sans qu'il soit nécessaire d'isoler le produit résultant de l'étape (e).

On porte à ébullition pendant 1 heure un mélange constitué de 1 g de l'acide obtenu précédemment, 5 $cm^3$ d'anhydride acétique, 2 $cm^3$ de triéthylamine et 5 $cm^3$ de sulfure de carbone. La solution est ensuite refroidie et le précipité obtenu est essoré, lavé à l'éther et recristallisé dans l'acétonitrile. Le point de fusion du dithiole mésoïonique obtenu est supérieur à 260°C.

• étape (g)

$2.10^{-3}$ mole du dérivé mésoïonique obtenu précédemment, en suspension dans 30 $cm^3$ d'acétone sec, est additionné à $8.10^{-3}$ mole du dérivé halogéné $Br(CH_2)_{17}CH_3$ et la suspension est portée au reflux pendant 16 heures. Après évaporation du solvant, on obtient le sel de dithiolium qui peut être utilisé directement pour l'étape suivante.

• étape (h)

Le sel de dithiolium obtenu à l'étape précédente est partiellement dissous dans 20 $cm^3$ d'éthanol. A la suspension refroidie par un bain de glace on ajoute sous agitation 100 mg de borohydrure de sodium. Après un temps de 10 mn, on ajoute 1 $cm^3$ d'acide chlorhydrique 12 N et après 30 mn à la température ambiante, le milieu est dilué par 60 $cm^3$ d'eau puis extrait à l'éther. Après évaporation du solvant on isole le produit obtenu (éthoxydithiole) sous forme d'un solide jaune dont le point de fusion est de 89°C.

• étape (i)

2.$10^{-3}$ mole de l'éthoxydithiole sec obtenu à l'étape précédente est porté à reflux pendant 1 heure dans 70 $cm^3$ de benzène sec en présence de 500 mg d'acide trichloroacétique. Après refroidissement, la solution est lavée à l'eau jusqu'à neutralité des eaux de lavage puis évaporée. Le TTF est obtenu sous forme d'un solide rouge, lavé à l'éther puis recristallisé dans de l'acétate d'éthyle. Le point de fusion du produit obtenu est compris entre 140 et 150°C. La figure 4 représente une molécule du TTF obtenu.

Le procédé général de synthèse permet l'élaboration des molécules selon l'invention, entre autres celles dont certaines caractéristiques sont regroupées dans les tableaux 1 et 2 placés en fin de description. Le tableau 1 concerne les dithioles mésoïoniques obtenus à l'issue de l'étape (f) et en fonction de certains radicaux $R^1$. Le tableau 2 concerne des molécules de TTF pour certaines compositions des radicaux $R^1$ et $R^2$. Ces deux tableaux indiquent la valeur des points de fusion F des différents produits ainsi que les données relatives aux caractéristiques RMN (protons). Pour le tableau 1, le solvant utilisé pour la caractérisation est du chloroforme deutéré. Pour le tableau 2, il s'agit soit de pyridine deutérée, soit de chloroforme deutéré.

Il faut noter que lorsque $R^1$ = $pCO_2HC_6H_4$ (ce qui est le cas de l'exemple décrit plus en détail ci-dessus), ce radical $R^1$ acquiert, lors de l'étape (e) un groupe CO qu'il perd lors de l'étape (h). C'est pour cette raison que le premier radical $R^1$ du tableau 1 apparaît sous la forme $pCH_3COOCOC_6H_4$.

La molécule de TTF de forme trans ou cis dont la synthèse est décrite ci-dessus est tout à fait adaptée à la formation d'un film monomoléculaire à la surface de l'eau, qui peut être ensuite transféré sur un substrat afin d'élaborer un film de Langmuir-Blodgett. Un exemple de fabrication d'un tel film est décrit ci-dessous.

Les molécules de TTF sont mises en solution $10^{-3}$ M dans du chloroforme. Cette solution est ensuite déposée à la surface d'une cuve d'eau dans laquelle est dissous du chlorure de calcium $10^{-3}$ M. Les molécules TTF forment alors une couche monomoléculaire qui peut être compressée pour obtenir un film monomoléculaire compact et stable à la surface de l'eau. Ce film est ensuite transféré et superposé sur un substrat.

Afin de rendre le film conducteur, on peut utiliser plusieurs méthodes de l'art connu. On peut faire réagir sur ce film des éléments chimiques accepteurs d'électrons tels que l'iode, le brome, le pentafluorure d'arsenic, le TCNQ, etc. On peut encore doper ces couches en dissolvant dans l'eau de la cuve les éléments accepteurs d'électrons et ainsi former directement à la surface de l'eau une couche monomoléculaire conductrice. On peut aussi préparer en solution dans le chloroforme le complexe constitué par du TTF et des éléments chimiques accepteurs d'électrons et ainsi former directement à la surface de l'eau une couche monomoléculaire conductrice.

A la fin de cette opération on obtient un film de Langmuir-Blodgett dont l'épaisseur peut comprendre une à plusieurs couches, chaque couche étant d'une épaisseur de l'ordre de 30 angströms. Ce film est conducteur de l'électricité dans les directions du plan du film et isolant dans une direction perpendiculaire à ce plan.

| $R^1$ | F (°C) | RMN |
|---|---|---|
| $pCH_3COOCOC_6H_4$ | > 260 | 1,82 (m, 6 H) ; 2,42 (s, 3 H) 3,62 (m, 4 H) ; 8,13 (m, 4 H) |
| $CH_3(CH_2)_{10}$ | 115 | 0,87 (t, 3 H) ; 1,25 (m, 18 H) 1,78 (m, 6 H) ; 2,70 (t, 2 H) 3,67 (m, 4 H) |
| $pClC_6H_4$ | 266 | 1,60 (m, 6 H) ; 3,60 (m, 4 H) 7,70 (m, 4 H) |

TABLEAU 1

| $R^1$ | $R^2$ | F (°C) | RMN |
|---|---|---|---|
| $pCO_2HC_6H_4$ | $(CH_2)_{17}CH_3$ | 140-150 | (pyridine deutérée) 0,94 (t, 6 H) ; 1,36 (m, 64 H) 2,92 (t, 4 H) ; 8,15 (m, 8 H) |
| $pClC_6H_4$ | $(CH_2)_{10}CO_2H$ | 138-140 | (Chloroforme deutéré) 1,23 (m, 32 H) ; 2,37 (t, 4 H) 2,62 (t, 4 H) ; 7,40 (m, 8 H) |
| $(CH_2)_{10}CH_3$ | $CH_2CO_2H$ | 90-95 | (pyridine deutérée) 0,86 (t, 6 H) ; 1,26 (m, 36 H) 2,75 (t, 4 H) ; 3,87 (s, 4 H) |
| $(CH_2)_{10}CH_3$ | $(CH_2)_{10}CO_2H$ | 108-112 | (pyridine deutérée) 0,90 (t, 6 H) ; 1,30 (m, 74 H) 2,65 (m, 12 H) |

TABLEAU 2

## Revendications

1. Composé organique du type tétrathiafulvalène, caractérisé en ce qu'il est rendu amphiphile par substitution de radicaux, le composé répondant aux formules suivantes :

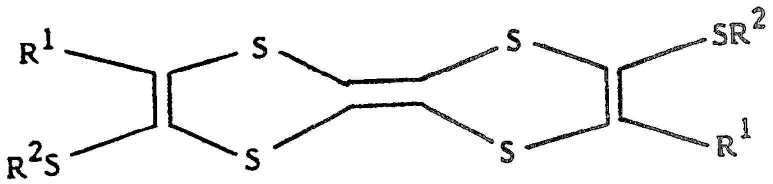

ou

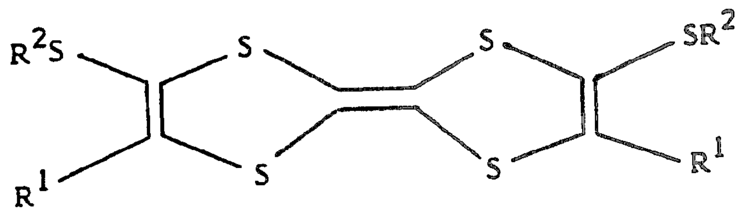

$R^1$ et $R^2$ répondant à l'une des conditions suivantes :

- $R^1$ est un groupe hydrophile et $R^2$ un groupe hydrophobe,
- $R^1$ est un groupe hydrophobe et $R^2$ un groupe hydrophile,
- $R^1$ est un groupe aromatique et $R^2$ est un groupe possédant une longue chaîne carbonée de nature hydrophobe et une extrémité hydrophile.

2. Composé selon la revendication 1, caractérisé en ce que $R^1$ est le groupe $pCO_2HC_6H_4$ et $R^2$ le groupe $(CH_2)_{17}CH_3$.

3. Composé selon la revendication 1, caractérisé en ce que $R^1$ est le groupe $pClC_6H_4$ et $R^2$ le groupe $(CH_2)_{10}CO_2H$.

4. Composé selon la revendication 1, caractérisé en ce que $R^1$ est le groupe $(CH_2)_{10}CH_3$ et $R^2$ le groupe $CH_2CO_2H$.

5. Composé selon la revendication 1, caractérisé en ce que $R^1$ est le groupe $(CH_2)_{10}CH_3$ et $R^2$ le groupe $(CH_2)_{10}CO_2H$.

6. Procédé de fabrication d'un composé organique selon l'une quelconque des revendications 1 à 5, caractérisé en qu'il comprend les étapes suivantes :

- étape (a) : préparation d'un éthylénique par action du malonitrile sur un aldéhyde comportant le radical $R^1$,
- étape (b) : préparation d'un époxyde par action de l'hypochlorite de sodium sur l'éthylénique obtenu à l'issue de l'étape (a),
- étape (c) : préparation d'un amide par action du dithiocarbamate de pipéridinium sur le produit obtenu à l'issue de l'étape (b),
- étape (d) : transformation en acide carboxylique du produit obtenu à l'issue de l'étape (c),
- étapes (e) et (f) : obtention d'un dithiole mésoïonique par action de l'anhydride acétique, de triéthylamine et de sulfure de carbone sur l'acide obtenu à l'issue de l'étape (d),
- étape (g) : préparation d'un sel de dithiolium par action d'un dérivé halogéné comportant le radical $R^2$ sur le produit obtenu à l'issue de l'étape (f),
- étape (h) : préparation d'un éthoxydithiole par action du borohydrure de sodium et de l'acide chlorhydrique sur le produit obtenu à l'issue de l'étape (g),
- étape (i) : obtention dudit composé organique par action de l'acide trichloroacétique sur le produit obtenu à l'issue de l'étape (h).

7. Film de Langmuir-Blodgett, caractérisé en ce qu'il est formé à partir du composé organique selon l'une quelconque des revendications 1 à 5, le film étant rendu conducteur par dopage.

**Ansprüche**

1. Organische Verbindung vom Tetrathiafulvalen-Typ, dadurch gekennzeichnet, daß sie durch Substitution mit Resten amphiphil gemacht worden ist, wobei die Verbindung den folgenden allgemeinen Formeln entspricht :

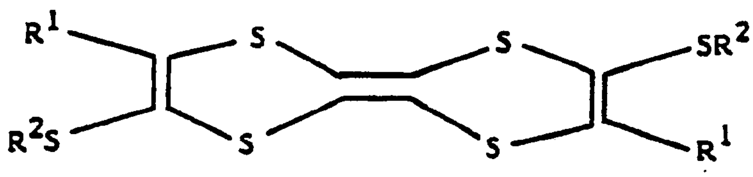

oder

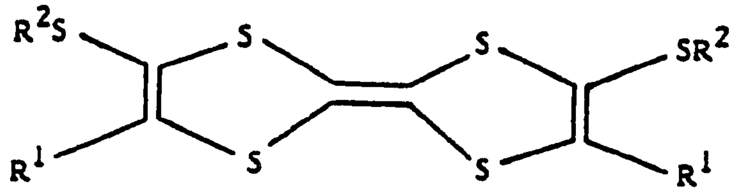

worin $R^1$ und $R^2$ einer der folgenden Bedingungen genügen :

– $R^1$ stellt eine hydrophile Gruppe dar und $R^2$ stellt eine hydrophobe Gruppe dar,

– $R^1$ stellt eine hydrophobe Gruppe dar und $R^2$ stellt eine hydrophile Gruppe dar,

– $R^1$ stellt eine aromatische Gruppe dar und $R^2$ stellt eine Gruppe dar, die eine hydrophobe lange Kohlenstoffkette und ein hydrophiles Ende aufweist.

2. Verbindung nach Anspruch 1, dadurch gekennzeichnet, daß $R^1$ die Gruppe p-$CO_2HC_6H_4$ und $R^2$ die Gruppe $(CH_2)_{17}CH_3$ darstellen.

3. Verbindung nach Anspruch 1, dadurch gekennzeichnet, daß $R^1$ die Gruppe p-$ClC_6H_4$ und $R^2$ die Gruppe $(CH_2)_{10}CO_2H$ darstellen.

4. Verbindung nach Anspruch 1, dadurch gekennzeichnet, daß $R^1$ die Gruppe $(CH_2)_{10}CH_3$ und $R^2$ die Gruppe $CH_2CO_2H$ darstellen.

5. Verbindung nach Anspruch 1, dadurch gekennzeichnet, daß $R^1$ die Gruppe $(CH_2)_{10}CH_3$ und $R^2$ die Gruppe $(CH_2)_{10}CO_2H$ darstellen.

6. Verfahren zur Herstellung einer organischen Verbindung nach einem der Ansprüche 1 bis 5, dadurch gekennzeichnet, daß es die folgenden Stufen umfaßt :

– Stufe (a) : Herstellung eines Ethylens durch Umsetzung von Malonitril mit einem den Rest $R^1$ aufweisenden Aldehyd,

– Stufe (b) : Herstellung eines Epoxids durch Umsetzung von Natriumhypochlorit mit dem in der Stufe (a) erhaltenen Ethylen,

– Stufe (c) : Herstellung eines Amids durch Umsetzung von Piperidiniumdithiocarbamat mit dem in der Stufe (b) erhaltenen Produkt,

– Stufe (d) : Umwandlung des in der Stufe (c) erhaltenen Produkts in eine Carbonsäure,

– Stufen (e) und (f) : Herstellung eines mesoionischen Dithiols durch Umsetzung von Essigsäureanhydrid, Triethylamin und Schwefelkohlenstoff mit der in der Stufe (d) erhaltenen Säure,

– Stufe (g) : Herstellung eines Dithioliumsalzes durch Umsetzung eines den Rest $R^2$ aufweisenden Halogenderivats mit dem in der Stufe (f) erhaltenen Produkt,

– Stufe (h) : Herstellung eines Ethoxydithiols durch Umsetzung von Natriumborhydrid und Chlorwasserstoffsäure mit dem in der Stufe (g) erhaltenen Produkt und

– Stufe (i) Herstellung der organischen Verbindung durch Umsetzung von Trichloressigsäure mit dem in der Stufe (h) erhaltenen Produkt.

7. Langmuir-Blodgett-Film, dadurch gekennzeichnet, daß er hergestellt wurde aus einer organischen Verbindung nach einem der Ansprüche 1 bis 5 und durch Dotierung elektrisch leitend gemacht wurde.

## Claims

1. An organic compound of the tetrathiafulvalene type, characterized in that it has been made amphiphilic by substitution with radicals, the compound corresponding to the following formulae :

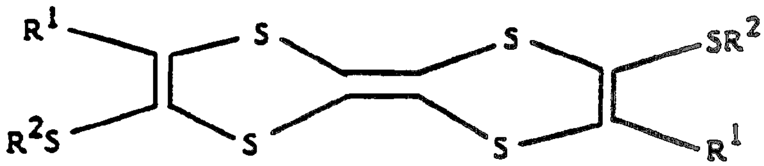

or

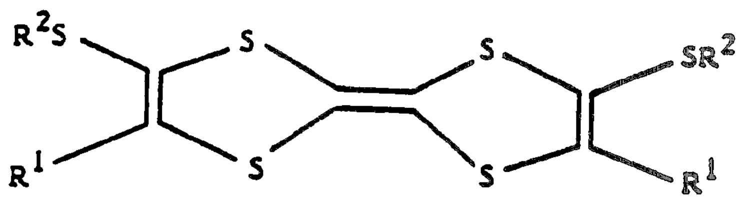

wherein $R^1$ and $R^2$ meet one of the following conditions :

– $R^1$ is a hydrophilic group and $R^2$ is a hydrophobic group,

– $R^1$ is a hydrophobic group and $R^2$ is a hydrophilic group, and

– $R^1$ is an aromatic group and $R^2$ is a group exhibiting a long chain of carbon atoms of hydrophobic nature and a hydrophilic end.

2. The compound according to claim 1, characterized in that $R^1$ is the group p-$CO_2HC_6H_4$ and $R^2$ is the group $(CH_2)_{17}CH_3$.

3. The compound according to claim 1, characterized in that $R^1$ is the group p-$ClC_6H_4$ and $R^2$ is the group $(CH_2)_{10}CO_2H$.

4. The compound according to claim 1, characterized in that $R^1$ is the group $(CH_2)_{10}CH_3$ and $R^2$ is the group $CH_2CO_2H$.

5. The compound according to claim 1, characterized in that $R^1$ is the group $(CH_2)_{10}CH_3$ and $R^2$ is the group $(CH_2)_{10}CO_2$ H.

6. A process for the preparation of an organic compound according to anyone of claims 1 to 5, characterized in that it comprises the following steps :

– step (a) : preparation of an ethylene by reacting malonitrile with an aldehyde containing the radical $R^1$,

– step (b) : preparation of an epoxide by reacting sodium hypochlorite with the ethylene obtained in step (a),

– step (c) : preparation of an amide by reacting piperidinium dithiocarbamate with the product obtained in step (b),

– step (d) : transformation of the product obtained in step (c) into a carboxylic acid,

– steps (e) and (f) : preparation of a mesoionic dithiole by reacting acetic anhydride, triethylamine and carbondisulfide with the acid obtained in step (d),

– step (g) : preparation of a dithiolium salt by reacting a halogen derivative containing the radical $R^2$ with the product obtained in step (f),

– step (h) : preparation of an ethoxydithiole by reacting sodium borohydride and hydrochloric acid with the product obtained in step (g), and

– step (i) : obtaining the said organic compound by reacting trichloroacetic acid with the product obtained in step (h).

7. Langmuir-Blodgett film, characterized in that it has been formed starting from the organic compound according to anyone of claims 1 to 5, the film being made conductive by doping.

FIG_1

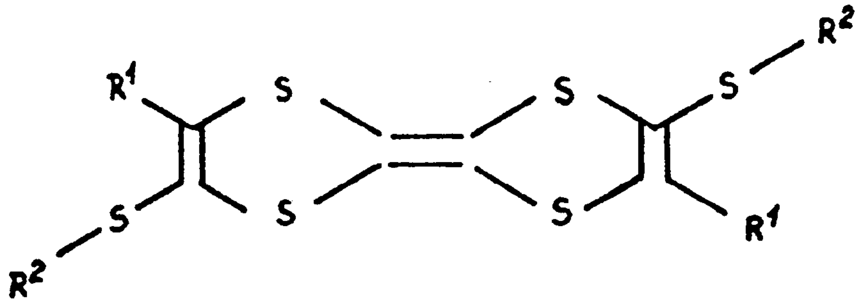

FIG_2

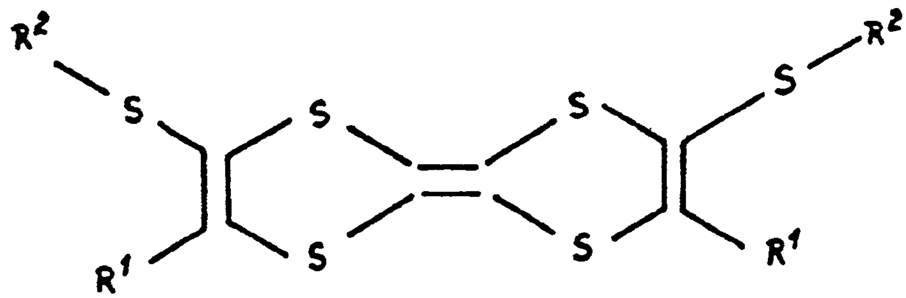

FIG_4

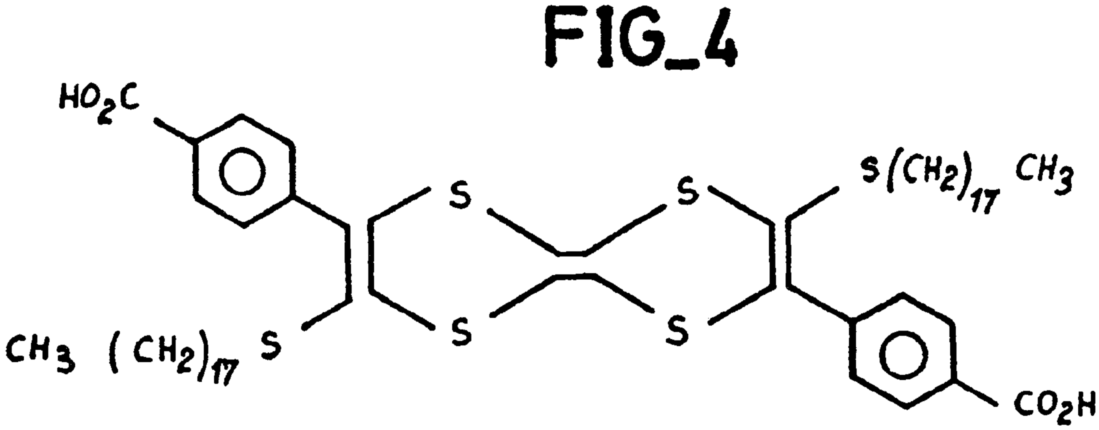

FIG_3

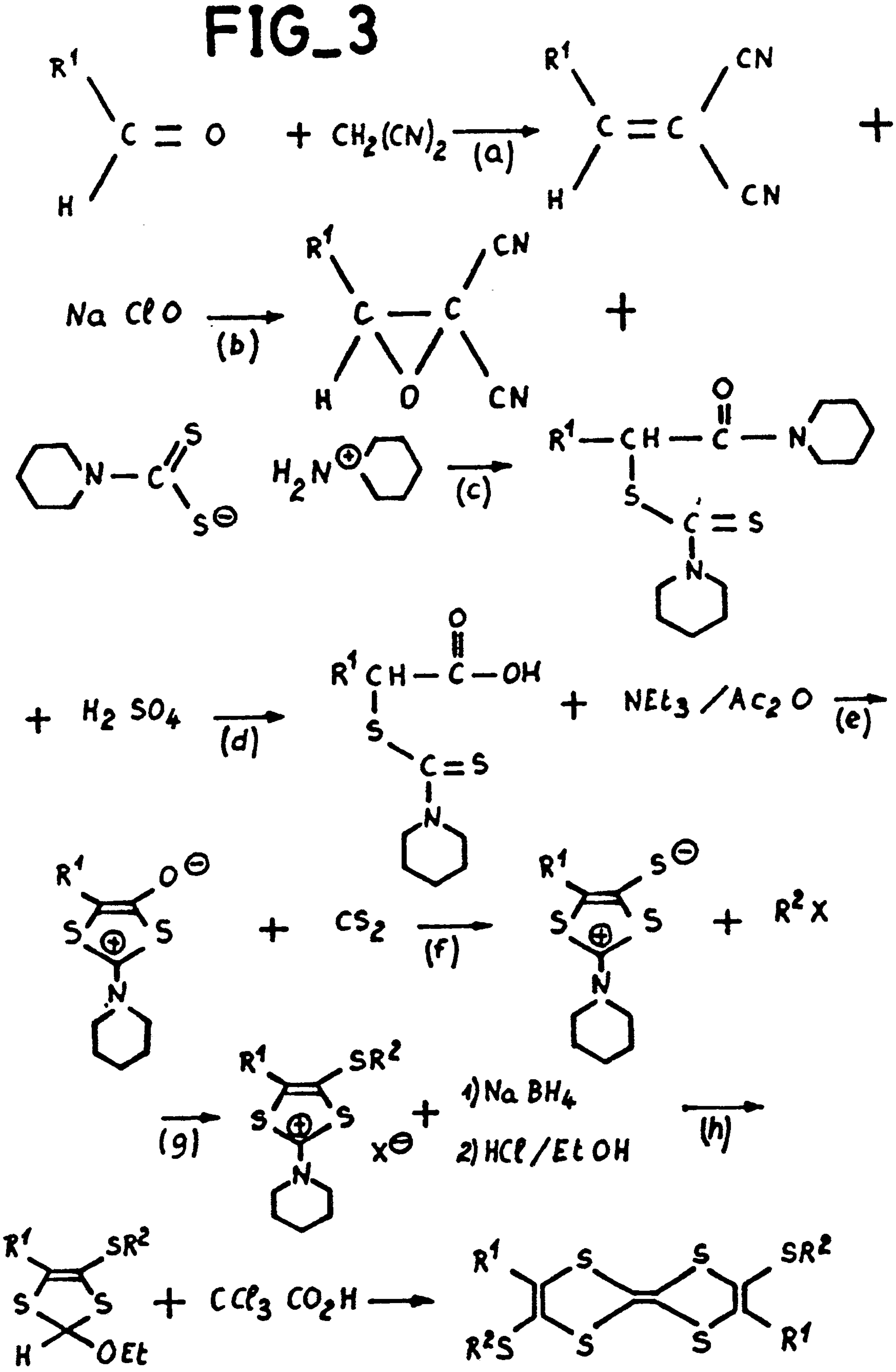

CH2(CN)2
(a)
Na ClO
(b)
(c)
H2 SO4
(d)
NEt3 / Ac2O
(e)
CS2
(f)
R2X
(g)
1) Na BH4
2) HCl / Et OH
(h)
CCl3 CO2H